# EUROPEAN PATENT APPLICATION

(11) **EP 4 282 519 A1**
(43) Date of publication of application: **29.11.2023**
(21) Application number: 23174901.1
(22) Date of filing: 23.05.2023
(51) Int. Cl.: B01F 33/84, B01F 33/30, B01F 25/433, B01F 101/21

(54) **APPARATUS FOR MANUFACTURING COSMETICS**

(30) Priority: 25.05.2022 KR 20220064024
(71) Applicant: Amorepacific Corporation, Seoul 04386 (KR)
(72) Inventor: Oh, Soojung, Yongin-si (KR); Han, Kyungsup, Yongin-si (KR); Baek, Heungsoo, Yongin-si (KR); Lee, Jeongin, Yongin-si (KR)
(74) Representative: Schön, Christoph

(57) **Abstract**

An aspect of the present disclosure may provide an apparatus for manufacturing cosmetics includes a storage part (100) capable of storing at least one fluid; and a main body (300) capable of accommodating a microfluidic chip (400) with a micro flow path formed therein, wherein the microfluidic chip is provided with at least one formulation (D1, D2) to be dissolved by the fluid, wherein the fluid flows along the micro flow path formed in the microfluidic chip and dissolves the formulation, and the fluid and the dissolved formulation are mixed in the microfluidic chip to form a cosmetic, and then discharged to the outside of the microfluidic chip.

## Description

### BACKGROUND

This disclosure relates to an apparatus for manufacturing cosmetics. Specifically, it relates to an apparatus for manufacturing cosmetics which is capable of manufacturing cosmetics using replaceable microfluidic chips.

Generally, cosmetics are used for beauty treatment, skin health, and the like, and in order to manufacture these cosmetics, a variety of apparatuses have been proposed. In particular, the market for customized cosmetics reflecting the user's skin condition and user needs is expanding, and various apparatuses for manufacturing such customized cosmetics are being proposed.

In general, cosmetics require an emulsification technique with which one liquid among two fluids which are not mixed with each other like water and oil can be dispersed in small particle form to be stably disposed within the other liquid. Such emulsification technique is widely used in the manufacture field of cosmetics, such as lotion, cream, essence, massage cream, cleansing cream, make-up base, foundation, eyeliner, mascara, or the like.

Conventional cosmetic manufacturing apparatuses have employed various devices for emulsifying fluids having opposite properties to form an emulsion. Accordingly, these cosmetic manufacturing apparatuses have had a problem in that it is difficult to manufacture them in a form that enables the customers to use them in customers' general homes because their volumes are large and their operating mechanisms are complicated.

In addition, the conventional cosmetic manufacturing apparatuses problematically take a very long time to manufacture cosmetics desired by a user.

### [Document of Related Art]

### [Patent Document]

Patent document 1: Korean Patent No. 10-0222000 (Registration Date: June 30, 1999)

### SUMMARY

The embodiments of the present disclosure are proposed to address the aforementioned problems, and intend to provide a miniaturized apparatus for manufacturing cosmetics which is capable of being used in general homes.

In addition, it is intended to provide an apparatus for manufacturing cosmetics which is capable of forming various emulsions according to user needs by replaceably providing various microfluidic chips including different cosmetic ingredients.

Moreover, it is intended to provide an apparatus for manufacturing cosmetics which is capable of shortening the manufacturing time of cosmetics.

According to an aspect of the present invention, there is provided an apparatus for manufacturing cosmetics comprising: a storage part capable of storing at least one fluid; and a main body capable of accommodating a microfluidic chip with a micro flow path formed therein, wherein the microfluidic chip is provided with at least one formulation to be dissolved by the fluid, and wherein the fluid flows along the micro flow path P formed in the microfluidic chip to dissolve the formulation and the fluid and the dissolved formulation are mixed in the microfluidic chip to form a cosmetic, and then discharged to the outside of the microfluidic chip.

Further, there is provided an apparatus for manufacturing cosmetics, further comprising a driving part which provides a pressure to cause the fluid and the formulation dissolved in the fluid to flow along the micro flow path P formed in the microfluidic chip.

Further, there is provided an apparatus for manufacturing cosmetics, further comprising a control part which controls the pressure applied from the driving part to the micro flow path P formed in the microfluidic chip, wherein the control part is capable of controlling the pressure applied to the micro flow path to differ according to the kind of the microfluidic chip.

Further, there is provided an apparatus for manufacturing cosmetics, wherein: the storage part includes a first storage part for storing a first fluid, and a second storage part for storing a second fluid; the microfluidic chip is provided with a first formulation to be dissolved by the first fluid, and a second formulation to be dissolved by the second fluid; and the first fluid, the dissolved first formulation, the second fluid, and the dissolved second formulation are mixed in the microfluidic chip to form a cosmetic, and then are discharged to the outside of the microfluidic chip.

Further, there is provided an apparatus for manufacturing cosmetics, wherein: the first fluid is provided as a hydrophilic fluid; the second fluid is provided as a hydrophobic fluid; the first formulation is formed in a solid phase or gel phase that can be dissolved in the hydrophilic fluid; and the second formulation is formed in a solid phase or gel phase that can be dissolved in the hydrophobic fluid.

Further, there is provided an apparatus for manufacturing cosmetics, wherein the cosmetic discharged to the outside of the microfluidic chip includes at least one of an emulsion in which the fluid and the dissolved formulation are mixed and emulsified, a lotion in which the fluid and the dissolved formulation are solubilized, and a mixed water in which the fluid and the dissolved formulation are mixed.

Further, there is provided an apparatus for manufacturing cosmetics, wherein the microfluidic chip includes: a dissolving portion in which a first fluid inlet through which the first fluid is introduced and a second fluid inlet through which the second fluid is introduced are formed, wherein the first formulation is disposed on a micro flow path P through which the first fluid flows, and the second formulation is disposed on a micro flow path P through which the second fluid flows; a mixing portion provided with a mixing flow path in which the first fluid, the dissolved first formulation, the second fluid, and the dissolved second formulation are mixed; and an emulsifying portion provided with a plurality of vortex forming flow paths which, in order to facilitate the mixing of the first fluid, the dissolved first formulation, the second fluid, and the dissolved second formulation to form an emulsion, form vortices in a flow thereof by changing the traveling direction of a fluid flowing therein.

Further, there is provided an apparatus for manufacturing cosmetics, wherein the width of the micro flow path P in which the first formulation is disposed or the width of the micro flow path P in which the second formulation is disposed is formed greater than the widths of other portions of the micro flow path formed in the microfluidic chip.

Further, there is provided an apparatus for manufacturing cosmetics, wherein a first coupling part connecting the first fluid inlet of the microfluidic chip and the first storage part is provided on the bottom side of the first storage part, and wherein a second coupling part connecting the second fluid inlet of the microfluidic chip and the second storage part is provided on the bottom side of the second storage part.

Further, there is provided an apparatus for manufacturing cosmetics, wherein the main body includes: a first main body in which a first insertion hole providing a space into which the microfluidic chip is inserted is formed; and a second main body in which a second insertion hole providing a space into which the storage part is inserted is formed.

Further, there is provided an apparatus for manufacturing cosmetics, wherein the microfluidic chip to be mounted on the main body is provided to be replaceable.

Further, there is provided an apparatus for manufacturing cosmetics, wherein a plurality of microfluidic chips are provided to be replaceable with respect to the main body, and wherein the structure of the micro flow path P formed in each of the plurality of microfluidic chips is formed in a different structure from each other.

Further, there is provided an apparatus for manufacturing cosmetics, wherein a plurality of microfluidic chips are provided to be replaceable with respect to the main body, and wherein the formulation disposed on each of the plurality of microfluidic chips is provided to have a different ingredient from each other.

The apparatus for manufacturing cosmetics according to the present embodiment has an advantage over conventional ones in that it can be miniaturized so that it can be used in general homes.

Also, the apparatus for manufacturing cosmetics can advantageously form various emulsions according to user needs by replaceably providing various microfluidic chips including different cosmetic ingredients.

Besides, the apparatus for manufacturing cosmetics has an advantage in shortening the manufacturing time of the cosmetics.

### BRIEF DESCRIPTION OF DRAWINGS

The above and other objects, features and advantages of this disclosure will become more apparent to those skilled in the art by describing exemplary embodiments thereof in detail with reference to the accompanying drawings, in which:
FIG. 1 is a diagram schematically showing a perspective view of an apparatus for manufacturing cosmetics 10 according to an embodiment of the present disclosure;
FIG. 2 is a diagram of the microfluidic chip 400 of the apparatus for manufacturing cosmetics 10 of FIG. 1 when viewed from the top;
FIG. 3 is an enlarged view of the dissolving portion 410 of the microfluidic chip 400 of FIG. 2;
FIG. 4 is an enlarged view of the mixing portion 420 of the microfluidic chip 400 of FIG. 2; and
FIG. 5 is an enlarged view of the emulsifying portion 430 of the microfluidic chip 400 of FIG. 2.

### DETAILED DESCRIPTION OF EXEMPLARY EMBODIMENTS

Hereinafter, specific exemplary embodiments of the present disclosure will be described in detail with reference to the accompanying drawings. Additionally, it is noted that in the description of the disclosure, the detailed description for known related configurations or functions may be omitted when it is deemed that such description may obscure essential points of the disclosure.

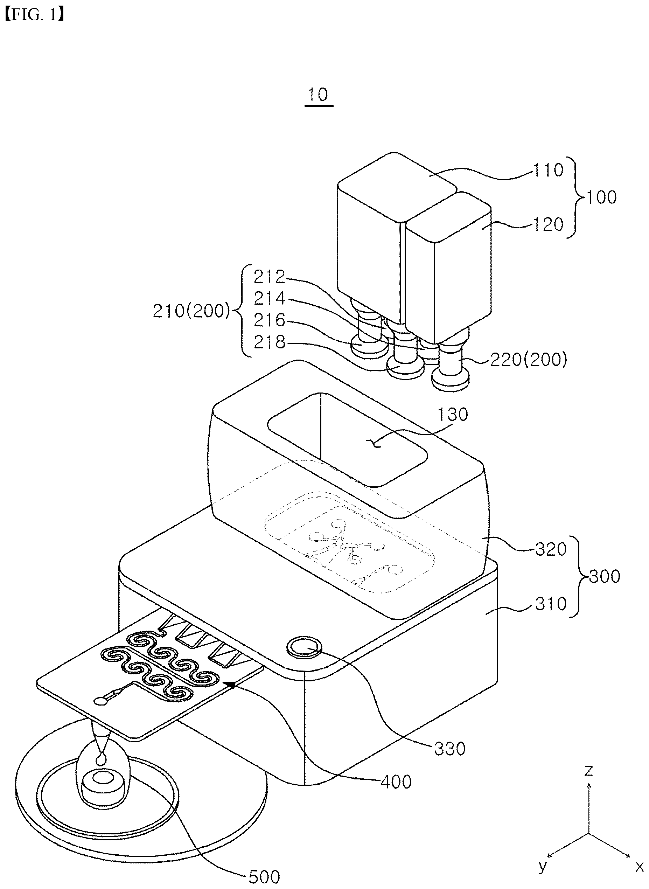

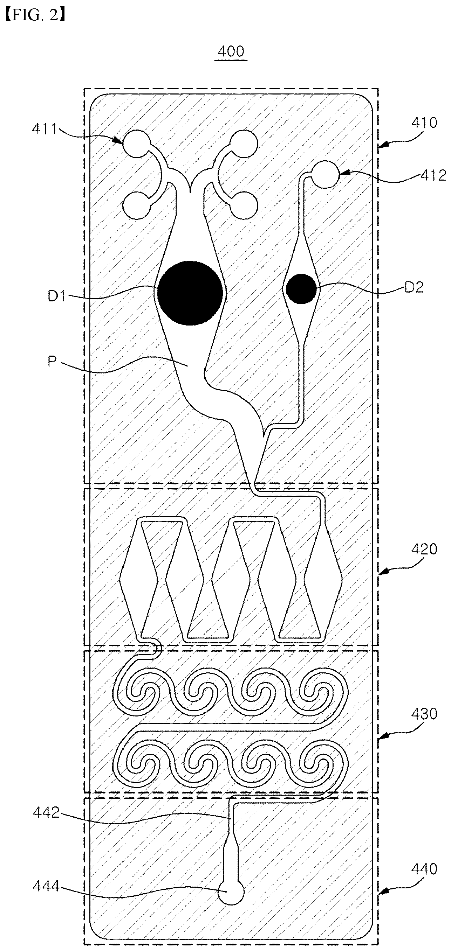

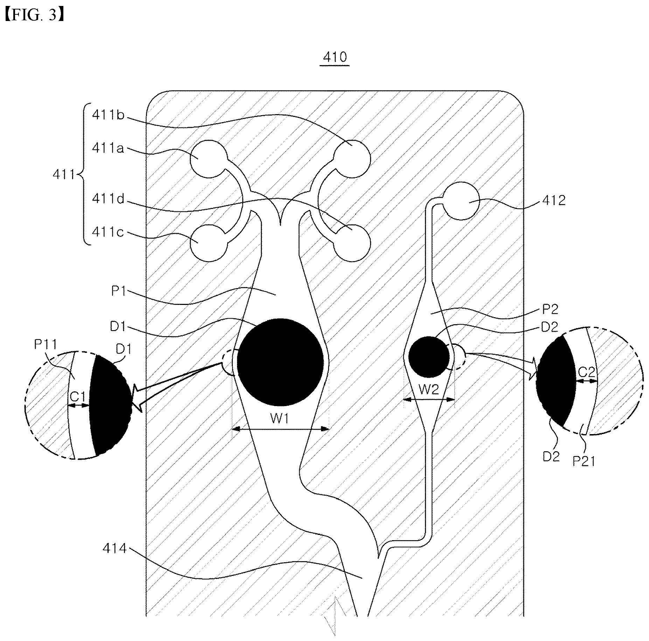

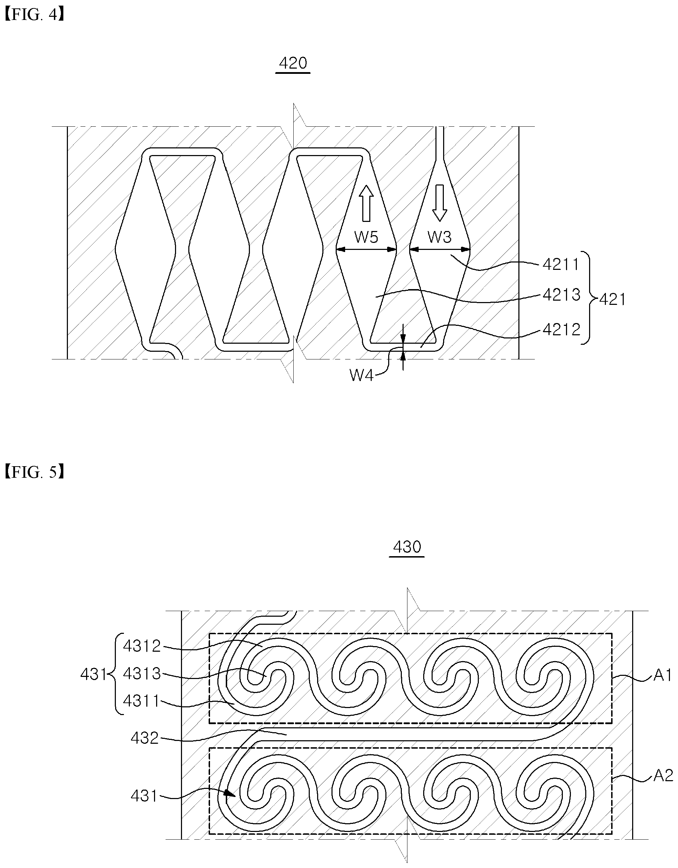

FIG. 1 is a diagram schematically showing a perspective view of an apparatus for manufacturing cosmetics 10 according to an embodiment of the present disclosure, FIG. 2 is a diagram of the microfluidic chip 400 of the apparatus for manufacturing cosmetics 10 of FIG. 1 when viewed from the top, FIG. 3 is an enlarged view of the dissolving portion 410 of the microfluidic chip 400 of FIG. 2, FIG. 4 is an enlarged view of the mixing portion 420 of the microfluidic chip 400 of FIG. 2, FIG. 5 is an enlarged view of the emulsifying portion 430 of the microfluidic chip 400 of FIG. 2.

Referring to FIGS. 1 to 5, the apparatus for manufacturing cosmetics 10 may include a storage part 100 capable of storing at least one fluid, and a main body 300 capable of accommodating the microfluidic chip 400 in which the micro flow path P is formed, wherein the microfluidic chip 400 may be fed with at least one formulation D1, D2 which can be dissolved by the fluid.

Here, the fluid flows along the micro flow path P formed in the microfluidic chip 400 to dissolve the formulations, and the fluid and the dissolved formulations are mixed in the microfluidic chip 400 to form a cosmetic, and then discharged from the microfluidic chip 400 to the outside.

Additionally, the apparatus for manufacturing cosmetics 10 may further include a driving part (not shown) providing a pressure by which the formulations flow along the micro flow path P formed in the microfluidic chip 400, and a control part (not shown) for controlling the pressure of the driving part (not shown) depending on the kind of the microfluidic chip 400.

Here, the classification of the kind of the microfluidic chip 400 can be determined depending on the amounts, ingredients, viscosity after the dissolving in the fluid, and the like of the formulations contained in the microfluidic chip, and information about the kind of the microfluidic chip 400 may be stored in advance in a database (not shown) embedded in the main body 300.

The apparatus for manufacturing cosmetics 10 of this embodiment may include a storage part for storing at least one fluid, and at least one formulation to be dissolved by the fluid and fed to the microfluidic chip 400.

It will be described herein by way of example as including a storage part for storing two kinds of fluids (first fluid and second fluid), respectively, and two kinds of formulations (first formulation D1 and second formulation D2) to be dissolved in the two kinds of fluids (first fluid and second fluid), respectively. However, the technical idea of the present disclosure is not limited to this, but may cover the case where it includes three or more kinds of fluids and three or more kinds of formulations to be dissolved in these fluids, respectively.

The apparatus for manufacturing cosmetics 10 of this embodiment may produce various cosmetics according to the kinds of the microfluidic chips 400.

The microfluidic chip 400 is replaceably provided to the apparatus for manufacturing cosmetics 10, and various kinds of formulations may be disposed on the microfluidic chip 400 to produce various cosmetics (e.g., emulsions).

Examples of the cosmetics produced in this embodiment may include, but not limited to, an emulsion in which a fluid and a dissolved formulation are mixed and emulsified, a lotion in which a fluid and a dissolved formulation are solubilized, and a mixed water in which a fluid and a dissolved formulation are simply mixed. In this embodiment, examples of the solubilized lotions may include, but not limited to, a skin lotion, an essence, and a perfume. This embodiment will be described by way of example under the condition that the cosmetic discharged from the microfluidic chip 400 is the emulsion.

In this embodiment, one kind of the microfluidic chip 400, in which the first and second formulations D1 and D2 are disposed on the micro flow path P, will be described by way of example.

The storage part 100 may include a first storage part 110 for storing a first fluid; and a second storage part 120 for storing a second fluid which is a different kind of fluid from the first fluid. In this connection, this embodiment will be described by way of example under the condition that water is provided as the first fluid as a hydrophilic fluid, and oil is provided as the second fluid as a hydrophobic fluid.

The first storage part 110 and the second storage part 120 may have their capacities corresponding to the amounts of the first fluid and the second fluid, respectively, which are completely discharged by one time operation. However, the technical idea of the present disclosure is not limited to this, but it may also be contemplated that the first storage part 110 and the second storage part 120 have their capacities corresponding to the amounts of the first fluid and the second fluid, respectively, which are completely discharged by multi-time operations.

Additionally, the first storage part 110 and the second storage part 120 may also be provided replaceably.

Below the storage part 100, a coupling part 200 may be provided, which connects the microfluidic chip 400 and the storage part 100.

Specifically, below the first storage part 110, a first coupling part 210 may be provided, which connects the first fluid inlet 411 of the microfluidic chip 400 with the first storage part 110 to deliver the first fluid stored in the first storage part 110 to the micro flow path P of the microfluidic chip 400.

Additionally, below the second storage part 120, a second coupling part 220 may be provided, which connects the second fluid inlet 412 of the microfluidic chip 400 with the second storage part 120 to deliver the second fluid stored in the second storage part 120 to the micro flow path P of the microfluidic chip 400.

The microfluidic chip 400 may be disposed below the coupling part 200.

The first coupling part 210 may include a first coupling flow path 212, a second coupling flow path 214, a third coupling flow path 216, and a fourth coupling flow path 218, which are connected to the first storage part 110.

Specifically, the first coupling flow path 212 may be coupled to the first upper inlet 411a of the microfluidic chip 400, the second coupling flow path 214 may be coupled to the second upper inlet 411b of the microfluidic chip 400, the third coupling flow path 216 may be coupled to the first lower inlet 411c of the microfluidic chip 400, and the fourth coupling flow path 218 may be coupled to the second lower inlet 411d of the microfluidic chip 400.

Also, the second coupling part 220 may be coupled to the second fluid inlet 412 of the microfluidic chip 400.

This embodiment will be described by way of example under the condition that the first fluid stored in the first storage part 110 is discharged to the micro flow path P of the dissolving portion 410 through the four coupling flow paths 112, 114, 116, and 118, and that the second fluid stored in the second storage part 120 is discharged to the micro flow path P of the dissolving portion 410 through one coupling flow path 220. However, the number of coupling flow paths of the present disclosure is not limited to this.

In this embodiment, the microfluidic chip 400 and the coupling part 200 coupled thereto may be disposed perpendicular to each other.

That is, the micro flow path P of the microfluidic chip 400 and the coupling part 200 may form an angle of 90 degrees.

For example, the micro flow path P of the microfluidic chip 400 may be disposed parallel to the bottom surface (in the X-Y plane) of the main body 300 when being inserted into the body 300, and the coupling part 200 is (300) may be disposed perpendicular to the bottom surface (in the Z-axis direction).

However, the technical idea of the present disclosure is not limited to this, but it may also be contemplated that the microfluidic chip 400 and the coupling part 200 are arranged in parallel to each other.

Specifically, in such case, the micro flow path P of the microfluidic chip 400 may be arranged in the direction of the gravity (in the Z-axis direction), so the fluid introduced into the dissolving portion 410 of the microfluidic chip 400 can be mixed and emulsified by gravity, and then be discharged to the outside through the discharge hole 444 of the discharge portion 440. In this case, the fluid may be mixed and emulsified by the gravity without a driving part.

The main body 300 may include a first main body 310 in which a first insertion hole (not shown) is formed to provide a space into which the microfluidic chip 400 is inserted; and a second main body 320 in which a second insertion hole 130 is formed to provide a space into which the storage part 100 is inserted.

Although this embodiment is described under the condition that the first main body 310 and the second main body 320 are separately installed, the first main body 310 and the second main body 320 may be integrally formed as one member.

The first insertion hole (not shown) into which the microfluidic chip 400 can be inserted may be formed in the first main body 310. Here, the first insertion hole (not shown) may be a space into which the microfluidic chip 400 can be inserted.

In addition, on one side of the main body 300, an operation button 330 may be disposed, which initiates an operation by which the fluid stored in the storage part 100 is moved to the microfluidic chip 400, and then mixed and emulsified with the formulation disposed on the microfluidic chip 400 to form an emulsion.

Additionally, a recognition part (not shown) capable of recognizing the kind of the microfluidic chip 400 may be provided in the first insertion hole (not shown) of the main body 300.

When the kind of the microfluidic chip 400 is recognized by the recognition part (not shown), the driving part (not shown) controlled by the control part (not shown) may apply a pressure corresponding to the kind of the microfluidic chip 400 to the micro flow path P.

The second insertion hole 130 into which the first storage part 110 and the second storage part 120 are inserted may be formed in the second main body 320.

In this embodiment, it is shown that the second insertion hole 130 is formed with one space. However, the technical idea of the present disclosure is not limited to this, but it may also be contemplated that the second insertion hole 130 is formed with a plurality of spaces so that they can accommodate the first storage part 110 and the second storage part 120, respectively.

The second main body 320 may be disposed on the upper side of the first main body 310.

The microfluidic chip 400 may be provided to be separable from the main body 300.

The microfluidic chip 400 may be provided commercially available separately, and different kinds of emulsions can be formed by exchanging only the different kinds of microfluidic chips 400.

As used herein, the emulsion may be understood as a cosmetic that can be applied to the user's skin.

The cosmetic manufactured by the apparatus for manufacturing cosmetics 10 of the present embodiment may include O/W (oil in water) emulsion which is manufactured by dispersing hydrophobic fluid, such as oil, uniformly in a small particle state in hydrophilic fluid, such as water, or W/O (water in oil) emulsion which is manufactured by dispersing hydrophilic fluid uniformly in a small particle state in hydrophobic fluid.

The microfluidic chip 400 detachably provided to the apparatus for manufacturing cosmetics 10 of the present embodiment may be provided in various ways so that emulsions having different efficacies can be produced.

For example, a plurality of microfluidic chips 400 may be provided to be replaceable with each other with respect to the main body 300, and formulations (e.g., the first formulation D1 and the second formulation D2) disposed on the plurality of microfluidic chips 400 may be provided to have different ingredients from each other.

In addition, the micro flow path P formed in each of the plurality of microfluidic chips 400 may be formed to have a different structure depending on the formulation disposed on each of the microfluidic chips 400.

In this embodiment, the structure of the micro flow path P of one kind of the microfluidic chip 400 as well as the formulation disposed therein will be described by way of example.

A plurality of formulations may be disposed on the micro flow path P of the microfluidic chip 400.

This embodiment is described by way of example under the condition that the first formulation D1 to be dissolved in a first fluid and the second formulation D2 to be dissolved in a second fluid are disposed on the microfluidic chip 400.

Here, the first formulation D1 may be a water-soluble material, and be formed in a solid (e.g., powder block) or gel phase.

Also, the second formulation D2 may be a oil-soluble material, and be formed in a solid (e.g., powder block) or gel phase.

However, the first formulation form D1 and the second formulation form D2 are not limited to this, but it may also be contemplated that they are a solid or gel phase with a liquid contained therein. In this case, the solid or gel phase surrounding the liquid is dissolved in the first fluid and the second fluid, respectively, so that the first fluid and the first formulation D1 can be mixed or the second fluid and the second formulation D2 can be mixed.

The micro flow paths P of the microfluidic chips 400 may be formed to have different structures according to the first and second formulations D1 and D2 included in the microfluidic chip 400.

For example, the first fluid and the first formulation D1 dissolved in the first fluid may include water, a polyol, a thickener, a surfactant, an auxiliary emulsifier, a moisturizing agent, a salt, a preservative, an aqueous phase (e.g. dissolved in water) functional ingredient, and the like.

Here, the polyol may be understood as a multifunctional alcohol having two or more hydroxyl groups (-OH) in a molecule. The polyol may include glycol having two hydroxyl groups, glycerol having three hydroxyl groups, and pentaerythritol having four hydroxyl groups.

The polyol may include at least one selected from the group consisting of ethylene glycol, propylene glycol, dipropylene glycol, 1,3-butylene glycol, diglycerin, 1,3-propanediol, glycerin, methylpropanediol, ethylhexanediol, 1,2-hexanediol, isoprene glycol, ethylalcohol, pentylene glycol, and isopentyldiol.

The thickener may include at least one selected from the group consisting of Gelatin, Gellan Gum, Guar Gum, Xanthan Gum, Locust Bean Gum, Alginic acid, Arabic Gum, Carrageenan, Pectin, Cellulose, Mannan, Carbomer, Sodium Polyacrylate, Polyacrylic Acid, Polyacrylate crosspolymer, Hydroxyethyl acrylate/Sodium acryloyldimethyl taurate Copolymer, Ammonium acryloyldimethyltaurate/vp copolymer, Sodium polyacryloyldimethyl taurate, Acrylate/C10-30 Alkyl acrylate Crosspolymer, Ammonium acryloyldimethyltaurate/beheneth-25 methacrylate crosspolymer, Poly vinyl alcohol, and glyceryl acrylate/acrylic acid copolymer.

Also, for example, the second fluid and the second formulation D2 dissolved in the second fluid may include oil, a surfactant, a co-emulsifier, a functional raw material dissolved in the oil, and the like.

Additionally, at least one of the first fluid, the first formulation D1 dissolved in the first fluid, the second fluid, and the second formulation D2 dissolved in the second fluid may include at least one of moisturizing agent, surfactant, thickener, and auxiliary emulsifier.

The moisturizing agent may include at least one selected from a group consisting of Trehalose, Fructose, Sucrose, Sorbitol, Maltitol, Panthenol, Raffinose, Urea, Betaine, Glycereth-26, Methyl Gluceth-20, PEG-8, PEG-32, PEG-75, PEG/PPG/POLYBUTYLENE GLYCOL-8/5/3 GLYCERIN, ethylene glycol, propylene glycol, dipropylene glycol, 1,3-butylene glycol, diglycerin, 1,3-propanediol, glycerin, methylpropanediol, ethylhexanediol, 1,2-hexanediol, isoprene glycol, ethylalcohol, pentylene glycol, and isopentyldiol.

The surfactant may include at least one selected from the group consisting of Sorbitan Monostearate, Sorbitan Sesquioleate, Polysorbate 20, Polysorbate 60, Polysorbate 80, PEG-40 Stearate, PEG-100 Stearate, PEG-60 Glyceryl Isostearate, PEG 75- Stearate, Ceteth-20, Steareth-20, Cetearyl Glucoside, Arachidyl Glucoside, Lauryl Glucoside, Sucrose Polystearate, Sucrose Laurate, Polyglyceryl-3 Methylglucose Distearate, Polyglyceryl-10 Stearate, Lecithin, Sodium Stearoyl Glutamate, Glyceryl Stearate Citrate, Potassium Cetyl Phosphate, Sodium Methyl Stearoyl Taurate, Inulin Lauryl Carbamate, Ceteareth-12, PEG-5 Rapeseed Sterol, Methoxy PEG-114/Polyepsion Caprolactone, Cetyl PEG/PPG 10/1 Dimethicone Triglyceride, PEG-10 Dimethicone, Lauryl PEG-9 Polydimethylsiloxyethyl Dimethicone, Polyglyceryl-2 Dipolyhydroxystearate, PEG-60 Hydrogenated Castor Oil, PEG-30 Hydrogenated Castor Oil, Octyldodeceth-16, Octyldodeceth-25, Bis-PEG-18 Methyl Ether Dimethyl Silane, Polyglyceryl-6 Caprylate, Polyglyceryl-4 Caprate.

The auxiliary emulsifier included in the first content may include at least one selected from a group consisting of Cetearyl Alcohol, Cetyl Alcohol, Stearyl Alcohol, Behenyl Alcohol, Glyceryl Stearate, Stearic Acid.

However, these are merely exemplary ingredients, and the kinds of raw materials included in the first fluid, the first formulation D1 dissolved in the first fluid, the second fluid, and the second formulation D2 dissolved in the second fluid are not limited to them. The first fluid, the first formulation D1 dissolved in the first fluid, the second fluid and the second formulation D2 dissolved in the second fluid may pass through the micro flow path P formed in the dissolving portion 410, the mixing portion 420, the emulsifying portion 430, and the discharge portion 440 of the microfluidic chip 400, and may be mixed and emulsified to be produced as an O/W emulsion or a W/O emulsion.

The microfluidic chip 400 may receive the first fluid from the first storage part 110 and the second fluid from the second storage part 120.

After the first fluid dissolves the first formulation D1 and the second fluid dissolves the second formulation D2 on the micro flow path P of the microfluidic chip 400, the first fluid, the dissolved first formulation D1, the second fluid, and the dissolved second formulation D2 may be mixed and emulsified to form an emulsion, and then may be discharged to the outside of the microfluidic chip 400.

Specifically, the type of emulsion (e.g., W/O emulsion or O/W emulsion) may be determined depending on the ratio of the first fluid and the second fluid supplied from the storage part 100 to the microfluidic chip 400.

The ratio of the first fluid and the second fluid supplied to the microfluidic chip 400 may be adjusted according to the discharge amounts of the first fluid and the second fluid discharged from the first storage part 110 and the second storage part 120, and the discharge amounts of the first fluid and the second fluid may be controlled by the control part (not shown).

For example, if the supply amount of water as the first fluid is greater than the supply amount of oil as the second fluid, an O/W emulsion is produced, while, if the supply amount of oil as the second fluid is greater than the supply amount of water as the first fluid, a W/O emulsion is produced.

The microfluidic chip 400 may be a plate in which the micro flow path P through which a fluid can flow is formed.

The microfluidic chip 400 may be a single layer provided in a flat plate shape, and the micro flow path P may be formed therein. In this way, the apparatus for manufacturing cosmetics 10 can be miniaturized by arranging the micro flow path P on one flat plate.

The micro flow path P formed inside the microfluidic chip 400 may have a rectangular cross section, and in this case, the average length of each side may be between 0.5 mm and 1 mm. When the micro flow path P has a circular cross section, the average length of the diameter may be 0.5 mm to 1 mm.

When the micro flow path P is formed inside the microfluidic chip 400, the flow rate of the fluid can be increased, so that the efficiency of mixing and emulsification can be increased.

Meanwhile, in this embodiment, the micro flow path P formed in the microfluidic chip 400 may substantially constitute a single layer path. The single layer path may be understood as a path in which height difference of flow paths is not involved in mixing and emulsification of each fluid or emulsification of the mixed fluid during the mixing and emulsification of fluid.

The single layer path of the present embodiment may correspond to a first micro flow path P1, a second micro flow path P2, a mixing flow path 421, a vortex forming flow path 431, and a discharge flow path 442 implemented on a single flat plate.

The microfluidic chip 400 may include the dissolving portion 410, the mixing portion 420, the emulsifying portion 430, and the discharge portion 440.

The dissolving portion 410 may include the first fluid inlet 411 through which the first fluid is introduced; the second fluid inlet 412 through which a second fluid is introduced; the first formulation D1 disposed in the first micro flow path P1 through which the first fluid flows and dissolved by the first fluid; the second formation D2 disposed in the second micro flow path P2 through which the second fluid flows and dissolved by the second fluid; and a confluence 414 where the first fluid, the dissolved first formulation D1, the second fluid, and the dissolved second formulation D2 meet.

Here, the width W1 of the micro flow path (first micro flow path P 1) in which the first formulation D1 is disposed and the width W2 of the micro flow path (second micro flow path P2) in which the second formulation D2 is disposed may be formed greater than the widths of the other portions of the micro flow path P.

As a result, the contact area between the first formulation D1 and the first fluid and the contact area between the second formulation D2 and the second fluid can be increased, so that the first formulation D1 and the second formulation D2 can be easily dissolved.

The first formulation D1 may be fixed on the first micro flow path P1, and the second formulation D2 may be fixed on the second micro flow path P2.

The first formulation D1 may be disposed in the first micro flow path P1, and the first fluid may pass through a first clearance flow path P11 formed between the first micro flow path P1 and the first formulation D1 (see FIG. 3) while dissolving the first formulation D1.

The second formulation D2 may be disposed in the second micro flow path P2, and the second fluid may pass through a second clearance flow path P21 formed between the second micro flow path P2 and the second formulation D2 while dissolving the second formulation D2.

The width C1 of the first clearance flow path P11 may be formed smaller than the width of the first micro flow path P1, and the width C2 of the second clearance flow path P21 may be formed smaller than the width of the second micro flow path P2.

As the first formulation D1 is dissolved in the first fluid, the width C1 may grow bigger, and as the second formulation D2 is dissolved in the second fluid, the width C2 may grow bigger.

As the width C1 of the first clearance flow path P11 and the width C2 of the second clearance flow path P21 become wider, the pressure applied to the micro flow path P of the microfluidic chip 400 by the driving part (not shown) may become higher. In this regard, the driving part (not shown) may be controlled by the control part (not shown).

That is, the clearance flow path through which a fluid can flow may be formed between the micro flow path P and the formulation, and as the formulation is dissolved by the fluid, the width of the clearance flow path can be increased, and as the width of the clearance flow path is increased, the pressure applied to the micro flow path P may be increased.

The mixing portion 420 may include a mixing flow path 421 in which the first fluid, the dissolved first formulation D1, the second fluid, and the dissolved second formulation D2 are mixed.

The mixing flow path 421 may be formed in a zigzag shape.

Specifically, the mixing flow path 421 may include a first mixing flow path 4211 extending from the micro flow path P formed in the dissolving portion 410 and extending in one direction; a second mixing flow path 4212 bent from the first mixing flow path 4211 by a preset angle (e.g., 90 degrees); and a third mixing flow path 4213 bent from the second mixing flow path 4212 by a preset angle (e.g., 90 degrees).

Here, the first mixing flow path 4211 and the third flow path 4213 may be formed in parallel to each other.

Here, the width W3 of the first mixing flow path 4211 and the width W5 of the third mixing flow path 4213 may increase and then decrease along the flow direction of the fluid, and the width W4 of the second mixing flow path 4212 may be formed to be constant.

The mixing portion 420 of this embodiment may include a plurality of first mixing flow paths 4211, a plurality of second mixing flow paths 4212, and a plurality of third mixing flow paths 4213. Specifically, the mixing portion 420 may be formed with three first mixing flow paths 4211, four second mixing flow paths 4212, and two third mixing flow paths 4213.

The emulsifying portion 430 may include a plurality of vortex forming flow paths 431 formed to form vortices in the flow by changing the traveling direction of the fluid flowing therein, so that the first fluid, the dissolved first formulation D1, the second fluid, and the dissolved second formulation D2 can be emulsified to form an emulsion.

In this regard, the emulsification may be understood as a technique with which one liquid among two fluids which are not mixed with each other like water and oil can be dispersed in small particle form to be stably disposed within the other liquid.

A plurality of vortex forming flow paths 431 may be continuously formed in a first region A1 of the emulsifying portion 430, and a plurality of vortex forming flow paths 431 may be continuously formed in a second region A2 disposed at a lower stage than the first region A1.

The vortex forming flow paths 431 formed in the first region A1 and the vortex forming flow paths 431 formed in the second region A2 may be connected through a connection flow path 432, and the connection flow path 432 may be formed as a straight-line flow path.

In this embodiment, the vortex forming flow path 431 is described by way of example as being configured to rotate the entering fluid in one direction (in this embodiment, anticlockwise based on the drawing) and then rotate it in the other direction (in this embodiment, clockwise based on the drawing).

Specifically, each of the vortex forming flow paths 431 may include a first turning flow path 4311 which guides the entering fluid so as to rotate in one direction, a second turning flow path 4312 which guides the fluid rotating in one direction so as to rotate in the other direction, and a direction conversion flow path 4313 which converts the rotating direction of the fluid between the first turning flow path 4311 and the second turning flow path 4312.

Here, as the first fluid, the dissolved first formulation D1, the second fluid, and the dissolved second formulation D2 pass through the plurality of vortex forming flow paths 431, the emulsification can proceed.

The external phase fluid (first fluid and dissolved first formulation D1, or second fluid and dissolved second formulation D2) and the internal phase fluid (second fluid and dissolved second formulation D2, or first fluid and dissolved first formulation D1) may become thinned or be broken due to the formation of a vortex while passing through the vortex forming flow path 431.

By repeating this process, finally, an emulsion in which the finely broken internal phase fluid is stably present within the external phase fluid can be formed.

The discharge portion 440 may extend from the emulsifying portion 430.

The discharge portion 440 may include a discharge flow path 442 extending from the vortex forming flow path 431 of the emulsifying portion 430, and the discharge hole 444 through which the produced emulsion is discharged.

The discharge flow path 442 may be formed to extend in one direction after being bent from the vortex-forming flow path 431 toward one side by a preset angle (e.g., 90 degrees).

The discharge flow path 442 may include a portion whose width increases as it goes from the vortex forming flow path 431 toward the discharge hole 444.

The discharge portion 440 may be formed at one end of the discharge flow path 442.

The above-described first micro flow path P1, second micro flow path P2, second fluid inlet 412, mixing flow path 421, vortex forming flow path 431, and discharge flow path 442 may be understood as the micro flow path P formed in the microfluidic chip 400.

In addition, the apparatus for manufacturing cosmetics 10 may further include a cosmetic container 500 disposed below the discharge hole 444 of the microfluidic chip 400.

The cosmetic container 500 may store the emulsion discharged from the discharge hole 444.

Also, the cosmetic container 500 may be optionally provided such that it is fixed to the microfluidic chip 400.

Specifically, the cosmetic container 500 may be disposed such that its inlet is aligned with the discharge hole 444 of the microfluidic chip 400, and the inlet of the cosmetic container 500 and the discharge hole 444 may be fixed to each other.

In this case, while the emulsion is being discharged from the microfluidic chip 400, the cosmetic container 500 and the microfluidic chip 400 may be maintained in a fixed state, and when the emulsion is completely discharged from the microfluidic chip 400, the cosmetic container 500 may be removed from the microfluidic chip 400.

Additionally, the apparatus for manufacturing cosmetics 10 may further include the driving part (not shown) which provides the pressure causing the fluid and the formulation dissolved in the fluid to flow along the micro flow path P formed in the microfluidic chip.

Specifically, the driving part may provide the pressure by which the first fluid, the dissolved first formulation D1, the second fluid, and the dissolved second formulation D2 flow along the micro flow path P.

The driving part may apply the pressure toward the micro flow path P formed from the first fluid inlet 411 and the second fluid inlet 412 to the discharge hole 444.

The driving part may apply the pressure from the first fluid inlet 411 and the second fluid inlet 412 to the discharge hole 444 along the micro flow path P by injecting air into the first fluid inlet 411 and the second fluid inlet 412.

In addition, the apparatus for manufacturing cosmetics 10 may further include the control part (not shown) which controls the pressure applied to the micro flow path P of the microfluidic chip 400 from the driving part.

The control part (not shown) may control the flow rate of the fluid flowing along the micro flow path P.

Specifically, the control part (not shown) may control the flow rate of the first fluid, the dissolved first formulation D1, the second fluid, and the dissolved second formulation D2, which are flowing along the mixing flow path 421 of the mixing portion 420, and may control the flow rate of the first fluid, the dissolved first formulation D1, the second fluid, and the dissolved second formulation D2, which are flowing along the vortex forming flow path 431 of the emulsifying portion 430.

The control part (not shown) may control the pressure applied to the micro flow path P differently depending on the kind of the microfluidic chip 400.

For example, when a first kind of microfluidic chip 400 is provided, the control part (not shown) may cause the driving part to apply a first pressure to the micro flow path P, and when a second kind of microfluidic chip 400 is provided, the control part (not shown) may cause the driving part to apply a second pressure to the micro flow path P.

Here, the first kind of microfluidic chip 400 may include a first formulation D1 including a first ingredient and a second formulation D2 including a second ingredient, and the second kind of microfluidic chip 400 may include a first formulation D1 including a third ingredient and a second formulation D2 including a fourth ingredient. Here, the first ingredient, the second ingredient, the third ingredient, and the fourth ingredient may be different from each other.

Additionally, the control part (not shown) controls the discharge amount of the first fluid stored in the first storage part 110, which is discharged to the microfluidic chip 400, and the discharge amount of the second fluid stored in the second storage part 120, which is discharged to the microfluidic chip 400.

Hereinafter, the operation process of the apparatus for manufacturing cosmetics 10 will be described.

A method for controlling an apparatus for manufacturing cosmetics according to this embodiment may include inserting one kind of microfluidic chip 400 into the main body 300 (step S1), applying a preset pressure corresponding to the kind of the microfluidic chip to the micro flow path P by the driving part (not shown) after the kind of the microfluidic chip 400 is recognized by the recognition part (not shown) (step S2), joining, at the confluence 414, the first fluid which has dissolved the first formulation D1 disposed in the dissolving portion 410 and the second fluid which has dissolved the second formulation D2 disposed in the dissolving portion 410 (step S3), mixing the first fluid, the dissolved first formulation D1, the second fluid, and the dissolved second formulation D2 by causing them to flow along the mixing flow path 421 formed in the mixing portion 420 (step S4), mixing the first fluid, the dissolved first formulation D1, the second fluid, and the dissolved second formulation D2 to form a cosmetic by causing them to flow along the vortex forming flow path 431 formed in the emulsifying portion 430 (step S5), and discharging the cosmetic to the outside along the discharge hole 444 of the discharge portion 440 (step S6).

While until now the apparatus for manufacturing cosmetics 10 and the microfludic chip 400 have been described with reference to examples of the disclosure as concrete embodiments, these are just exemplary embodiments, and the present disclosure should be construed in the broadest manner based on the fundamental technical ideas disclosed herein, rather than being limited to them. By combining or replacing a part or parts of embodiments disclosed herein, a person having ordinary skill in the art may practice an embodiment which is not explicitly described herein, and however, it should be noted that it shall not depart from the scope of the patent right of this disclosure. Besides, a person having ordinary skill in the art may easily change or modify embodiments disclosed herein based on this disclosure, and however, it is obvious that such changes or modifications also fall within the scope of the patent right of this disclosure.

The following is a list of the above-described embodiments.

Item 1 may provide an apparatus for manufacturing cosmetics, the apparatus including: a storage part capable of storing at least one fluid; and a main body capable of accommodating a microfluidic chip with a micro flow path formed therein, wherein the microfluidic chip is provided with at least one formulation to be dissolved by the fluid, wherein the fluid flows along the micro flow path formed in the microfluidic chip and dissolves the formulation, and wherein the fluid and the dissolved formulation are mixed in the microfluidic chip to form a cosmetic (for example, an emulsion in which the fluid and the dissolved formulation are mixed and emulsified, a lotion in which the fluid and the dissolved formulation are solubilized, and a mixed water in which the fluid and the dissolved formulation are mixed), and then discharged to the outside of the microfluidic chip.

Item 2 may provide the apparatus for manufacturing cosmetics of Item 1, further comprising a driving part which provides a pressure to cause the fluid and the formulation dissolved in the fluid to flow along the micro flow path formed in the microfluidic chip.

Item 3 may provide the apparatus for manufacturing cosmetics of Item 1 or Item 2, further comprising a control part which controls a pressure applied from the driving part to the micro flow path formed in the microfluidic chip, wherein the control part is capable of controlling the pressure applied to the micro flow path to differ according to the kind of the microfluidic chip.

Item 4 may provide the apparatus for manufacturing cosmetics of Items 1 to 3, wherein the storage part includes a first storage part for storing a first fluid, and a second storage part for storing a second fluid, wherein the microfluidic chip is provided with a first formulation to be dissolved by the first fluid, and a second formulation to be dissolved by the second fluid, and wherein the first fluid, the dissolved first formulation, the second fluid, and the dissolved second formulation are mixed in the microfluidic chip to form a cosmetic, and then are discharged to the outside of the microfluidic chip.

Item 5 may provide the apparatus for manufacturing cosmetics of Items 1 to 4, wherein the first fluid is provided as a hydrophilic fluid, wherein the second fluid is provided as a hydrophobic fluid, wherein the first formulation is formed in a solid phase or gel phase that can be dissolved in the hydrophilic fluid, and wherein the second formulation is formed in a solid phase or gel phase that can be dissolved in the hydrophobic fluid.

Item 6 may provide the apparatus for manufacturing cosmetics of Items 1 to 5, wherein the microfluidic chip includes a dissolving portion in which a first fluid inlet through which the first fluid is introduced and a second fluid inlet through which the second fluid is introduced are formed, wherein the first formulation is disposed on a micro flow path through which the first fluid flows, and the second formulation is disposed on a micro flow path through which the second fluid flows; a mixing portion provided with a mixing flow path in which the first fluid, the dissolved first formulation, the second fluid, and the dissolved second formulation are mixed; and an emulsifying portion provided with a plurality of vortex forming flow paths which, in order for the first fluid, the dissolved first formulation D1, the second fluid, and the dissolved second formulation D2 to be emulsified and form an emulsion, form vortices in a flow by changing the traveling direction of a fluid flowing therein.

Item 7 may provide the apparatus for manufacturing cosmetics of Items 1 to 6, wherein the mixing flow path includes: a first mixing flow path extending in one direction from a micro flow path formed in the dissolving portion; a second mixing flow path bent from the first mixing flow path by a preset angle; and a third mixing flow path bent from the second mixing flow path by a predetermined angle, and wherein the first mixing flow path and the third mixing flow path are formed in parallel to each other.

Item 8 may provide the apparatus for manufacturing cosmetics of Items 1 to 7, wherein at least one of the width of the first mixing flow path and the width of the third mixing flow path increases and then decreases along a fluid flow direction therein.

Item 9 may provide the apparatus for manufacturing cosmetics of Items 1 to 8, wherein a plurality of the vortex forming flow paths are continuously formed in a first region of the emulsifying portion, and a plurality of the vortex forming flow paths are continuously formed in a second region disposed at a lower stage than the first region.

Item 10 may provide the apparatus for manufacturing cosmetics of Items 1 to 9, wherein each of the vortex forming flow paths includes a first turning flow path which guides an entering fluid to be rotated in one direction; a second turning flow path which guides the fluid rotating in the one direction to be rotated in another direction; and a direction conversion flow path which changes a rotational direction of fluid between the first turning flow path and the second turning flow path.

Item 11 may provide the apparatus for manufacturing cosmetics of Items 1 to 10, wherein the width of the micro flow path in which the first formulation is disposed or the width of the micro flow path in which the second formulation is disposed is formed greater than the widths of other portions of the micro flow path formed in the microfluidic chip.

Item 12 may provide the apparatus for manufacturing cosmetics of Items 1 to 11, wherein a first coupling part connecting the first fluid inlet of the microfluidic chip and the first storage part is provided on the bottom side of the first storage part, and wherein a second coupling part connecting the second fluid inlet of the microfluidic chip and the second storage part is provided on the bottom side of the second storage part.

Item 13 may provide the apparatus for manufacturing cosmetics of Items 1 to 12, wherein the main body includes a first main body in which a first insertion hole providing a space into which the microfluidic chip is inserted is formed; and a second main body in which a second insertion hole providing a space into which the storage part is inserted is formed.

Item 14 may provide the apparatus for manufacturing cosmetics of Items 1 to 13, wherein the microfluidic chip to be mounted on the main body is provided to be replaceable.

Item 15 may provide the apparatus for manufacturing cosmetics of Items 1 to 14, wherein a plurality of microfluidic chips are provided to be replaceable with respect to the main body, and wherein the structure of the micro flow path formed in each of the plurality of microfluidic chips is formed in a different structure from each other.

Item 16 may provide the apparatus for manufacturing cosmetics of Items 1 to 15, wherein a plurality of microfluidic chips are provided to be replaceable with respect to the main body, and the formulation disposed on each of the plurality of microfluidic chips is provided to have a different ingredient from each other.

### [Reference Sign List]

10 : apparatus for manufacturing cosmetics
100 : storage part
110 : first storage part
120 : second storage part
130 : second insertion hole
200 : coupling part
210 : first coupling part
212 : first coupling flow path
214 : second coupling flow path
216 : third coupling flow path
218 : fourth coupling flow path
220 : second coupling part
300 : main body
310 : first main body
320 : second main body
330 : operation button
400 : microfluidic chip
410 : dissolving portion
411 : first fluid inlet
411a : first upper inlet
411b : second upper inlet
411c : first lower inlet
411d : second lower inlet
412 : second fluid inlet
414 : confluence
420 : mixing portion
421 : mixing flow path
4211 : first mixing flow path
4212 : second mixing flow path
4213 : third mixing flow path
430 : emulsifying portion
431 : vortex forming flow path
4311 : first turning flow path
4312 : second turning flow path
4313 : direction conversion flow path
432 : connection flow path
440 : discharge portion
442 : discharge flow path
444 : discharge hole
500 : cosmetic container

## Claims

1. An apparatus for manufacturing cosmetics comprising:
a storage part 100 capable of storing at least one fluid; and
a main body 300 capable of accommodating a microfluidic chip 400 with a micro flow path formed therein,
wherein the microfluidic chip 400 is provided with at least one formulation D1, D2 to be dissolved by the fluid, and
wherein the fluid flows along the micro flow path P formed in the microfluidic chip 400 to dissolve the formulation D1, D2, and the fluid and the dissolved formulation are mixed in the microfluidic chip to form a cosmetic, and then discharged to the outside of the microfluidic chip.

2. The apparatus for manufacturing cosmetics of claim 1, further comprising a driving part which provides a pressure to cause the fluid and the formulation dissolved in the fluid to flow along the micro flow path P formed in the microfluidic chip.

3. The apparatus for manufacturing cosmetics of claim 2, further comprising a control part which controls the pressure applied from the driving part to the micro flow path P formed in the microfluidic chip 400,
wherein the control part is capable of controlling the pressure applied to the micro flow path to differ according to the kind of the microfluidic chip 400.

4. The apparatus for manufacturing cosmetics of claim 1, wherein:
the storage part 100 includes a first storage part 110 for storing a first fluid, and a second storage part 120 for storing a second fluid;
the microfluidic chip 400 is provided with a first formulation D1 to be dissolved by the first fluid, and a second formulation D2 to be dissolved by the second fluid; and
the first fluid, the dissolved first formulation, the second fluid, and the dissolved second formulation are mixed in the microfluidic chip 400 to form a cosmetic, and then are discharged to the outside of the microfluidic chip.

5. The apparatus for manufacturing cosmetics of claim 4, wherein:
the first fluid is provided as a hydrophilic fluid;
the second fluid is provided as a hydrophobic fluid;
the first formulation is formed in a solid phase or gel phase that can be dissolved in the hydrophilic fluid; and
the second formulation is formed in a solid phase or gel phase that can be dissolved in the hydrophobic fluid.

6. The apparatus for manufacturing cosmetics of claim 1, wherein the cosmetic discharged to the outside of the microfluidic chip 400 includes at least one of an emulsion in which the fluid and the dissolved formulation are mixed and emulsified, a lotion in which the fluid and the dissolved formulation are solubilized, and a mixed water in which the fluid and the dissolved formulation are mixed.

7. The apparatus for manufacturing cosmetics of claim 4, wherein the microfluidic chip 400 includes:
a dissolving portion 410 in which a first fluid inlet 411 through which the first fluid is introduced and a second fluid inlet 412 through which the second fluid is introduced are formed, wherein the first formulation is disposed on a micro flow path P through which the first fluid flows, and the second formulation is disposed on a micro flow path P through which the second fluid flows;
a mixing portion 420 provided with a mixing flow path 421 in which the first fluid, the dissolved first formulation, the second fluid, and the dissolved second formulation are mixed; and
an emulsifying portion 430 provided with a plurality of vortex forming flow paths 431 which, in order to facilitate the mixing of the first fluid, the dissolved first formulation, the second fluid, and the dissolved second formulation to form an emulsion, form vortices in a flow thereof by changing the traveling direction of a fluid flowing therein.

8. The apparatus for manufacturing cosmetics of claim 4, wherein the width of the micro flow path P in which the first formulation is disposed or the width of the micro flow path P in which the second formulation is disposed is formed greater than the widths of other portions of the micro flow path formed in the microfluidic chip.

9. The apparatus for manufacturing cosmetics of claim 4, wherein a first coupling part 210 connecting the first fluid inlet 411 of the microfluidic chip 400 and the first storage part 110 is provided on the bottom side of the first storage part, and
wherein a second coupling part 220 connecting the second fluid inlet 412 of the microfluidic chip 400 and the second storage part 120 is provided on the bottom side of the second storage part 120.

10. The apparatus for manufacturing cosmetics of claim 1, wherein the main body 300 includes:
a first main body 310 in which a first insertion hole providing a space into which the microfluidic chip 400 is inserted is formed; and
a second main body 320 in which a second insertion hole providing a space into which the storage part 100 is inserted is formed.

11. The apparatus for manufacturing cosmetics of claim 1, wherein the microfluidic chip 400 to be mounted on the main body 300 is provided to be replaceable.

12. The apparatus for manufacturing cosmetics of claim 1, wherein a plurality of microfluidic chips 400 are provided to be replaceable with respect to the main body, and
wherein the structure of the micro flow path P formed in each of the plurality of microfluidic chips 400 is formed in a different structure from each other.

13. The apparatus for manufacturing cosmetics of claim 1, wherein a plurality of microfluidic chips 400 are provided to be replaceable with respect to the main body, and
wherein the formulation D1, D2 disposed on each of the plurality of microfluidic chips 400 is provided to have a different ingredient from each other.
